# EUROPEAN PATENT SPECIFICATION

(11) **EP 0 367 220 B1**
(45) Date of publication and mention of the grant of the patent: **07.01.1998**
(21) Application number: 89120186.5
(22) Date of filing: 31.10.1989
(51) Int. Cl.: C07K 1/00, C07K 1/16, C07K 1/18

(54) **Albumin preparation and process for preparing the same**
Albumin enthaltende Zusammensetzung und Verfahren zu ihrer Herstellung
Préparation d'albumine et son procédé d'obtention

(30) Priority: 31.10.1988 JP 277082/88; 28.04.1989 JP 111681/89
(43) Date of publication of application: 09.05.1990
(73) Proprietor: THE GREEN CROSS CORPORATION, Osaka-shi Osaka (JP)
(72) Inventor: Matsuoka, Yasushi, c/o The Green Cross Corp., Hirakata-shi, Osaka (JP); Hase, Shinichirou, c/o The Green Cross Corp., Hirakata-shi, Osaka (JP); Takechi, Kazuo, c/o The Green Cross Corp., Hirakata-shi, Osaka (JP); Tomioka, Shinji, c/o The Green Cross Corp., Takatsuki-shi, Osaka (JP); Yokoyama, Kazumasa, c/o The Green Cross Corp., Hirakata-shi, Osaka (JP)
(74) Representative: Hansen, Bernd, Dr. Dipl.-Chem.

(56) References cited:
- EP-A- 0 121 468
- EP-A- 0 144 714
- DE-A- 2 537 123
- FR-A- 2 327 256
- US-A- 4 043 997
- US-A- 4 075 197
- US-A- 4 228 154
- CHEMICAL ABSTRACTS, vol. 108, no. 25, 20 June 1988, Columbus, OH (US); C.H. MARSDEN et al., p. 283, no. 218559j

## Description

### FIELD OF THE INVENTION

This invention relates to an albumin preparation and a process for preparing the same. More particularly, it relates to a serum albumin preparation having a reduced content of agglomerates and a reduced content of contaminating proteins and to a process for preparing the same.

### BACKGROUND OF THE INVENTION

Serum albumin is present in blood plasma in a proportion higher than any other plasma protein and functions to maintain an osmotic pressure or to carry nutrients, metabolites, etc. as a combination therewith in blood.

Preparations containing serum albumin are used in the treatment of hypoalbuminemia, hemorrhagic shock, etc. caused by albumin depletion or reduction of albumin biosynthesis. To deactivate viruses which may be incorporated into the albumin preparations, a heat treatment of an albumin-containing aqueous solution is generally carried out. It is known that commercially available albumin preparations obtained from the thus treated albumin aqueous solution contain agglomerates (hereinafter referred to as polymers as such agglomerates are commonly called) which have been formed during preparation. From the fact that no substantial polymers are found before the above-described heat treatment, it is believed that heat-labile proteins are partially denatured by the heat treatment to form polymers. Considering that the commercially available albumin preparations have been widely used with safety, these polymers are not regarded particularly harmful to human bodies. However, it is desired that the preparations contain no polymers because the polymers are heat denaturation products.

Further, albumin preparations also contain α₁-acid glycoprotein (hereinafter referred to as α₁-AGP), having an immunosuppressive activity, as an impurity. α₁-AGP is a protein somewhat similar to albumin in physicochemical properties and, therefore, it is difficult to efficiently separate α₁-AGP from albumin by commonly employed means, such as fractionation. Hence, there is a fear that usual albumin preparations contain residual α₁-AGP having an immunosuppressive activity, and it is desired to remove α₁-AGP from the preparations to the extent possible.

FR 2327256 discloses albumin having a purity of not less than 96% and a polymer content of not more than 3.5% D1 does not describe the possibility of contamination with haptoglobin, α ₁-AGP and prealbumin. D1 also discloses an anion exchanger treatment at pH 4.5 to 4.9 for purification of albumin.

EP 121 468 discloses that albumin having a purity of 100% can be prepared. However, it is described in Example 5 that α₁-anti-trypsin and haptoglobin were detected in the purified albumin. D2 also discloses an anion exchanger treatment at pH 4.4 to 4.8 for purification of albumin.

DE 2537123 discloses an anion exchanger treatment at pH 6.5 for purification of albumin, in which DEAE-cellulose named Protion is used as an anion exchanger.

US 4228154 discloses the preparation of albumin having a maximal purity of 98.9%, but there is no description of a polymer content and the possibility of contamination with haptoglobin, α₁-AGP and prealbumin. D4 also discloses an anion exchanger treatment at pH 4.7 for purification of albumin.

US 4075197 discloses the preparation of albumin having a purity of 95.2%, but does not disclose a polymer content and contamination with haptoglobin, α₁-AGP and prealbumin. D5 also discloses an anion exchanger treatment for purification of albumin, which comprises allowing albumin to be adsorbed by an anion exchanger at pH 5.0 to 5.5 and eluting the albumin at pH 3.5 to 4.5.

J.M. Curling (Methods of Plasma Protein Fractionation, Ed. J. M. Curling, Acedemic Press, London, 1980, pages 77-91) discloses an anion exchanger treatment for purification of albumin, which comprises allowing albumin to be adsorbed by an anion exchanger at pH 5.2 and eluting the albumin at pH 4.5.

### SUMMARY OF THE INVENTION

An object of this invention is to provide an albumin preparation having a reduced polymer content and a reduced α₁-AGP content.

Another object of this invention is to provide a process for preparing the above-described albumin preparation.

As a result of extensive investigations, the inventors have found that formation of polymers is attributed to a polymer-forming factor (agglomerate-forming factor) present in albumin and that the polymer-forming factor is heat-labile contaminating proteins mainly comprising haptoglobin. It has also been found that an albumin preparation having reduced content of not only polymers but also of α₁-AGP can be obtained, even if a heat treatment is carried out, by removing the polymer-forming factor from albumin. The present invention has been completed based on these findings.

That is, the present invention relates to an albumin preparation having a polymer content of not more than 3% by weight based on the serum albumin content and an α₁-AGP content of less than 4 mg/dℓ in a 25% adjusted serum albumin solution.

The present invention also relates to a process for preparing an albumin preparation in which a polymer-forming factor comprising proteins having an isoelectric point lower than that of albumin and mainly comprising haptoglobin is removed which comprises subjecting a serum albumin aqueous solution to a step removing said polymer forming factor present in the solution, said step being carried out by at least one of ion exchange separation at a pH between 4.9 to 5.5 using an ion exchanger and affinity chromatography, and then subjecting the solution to a heat treatment sufficient to inactivate virus.

### BRIEF DESCRIPTION OF THE DRAWINGS

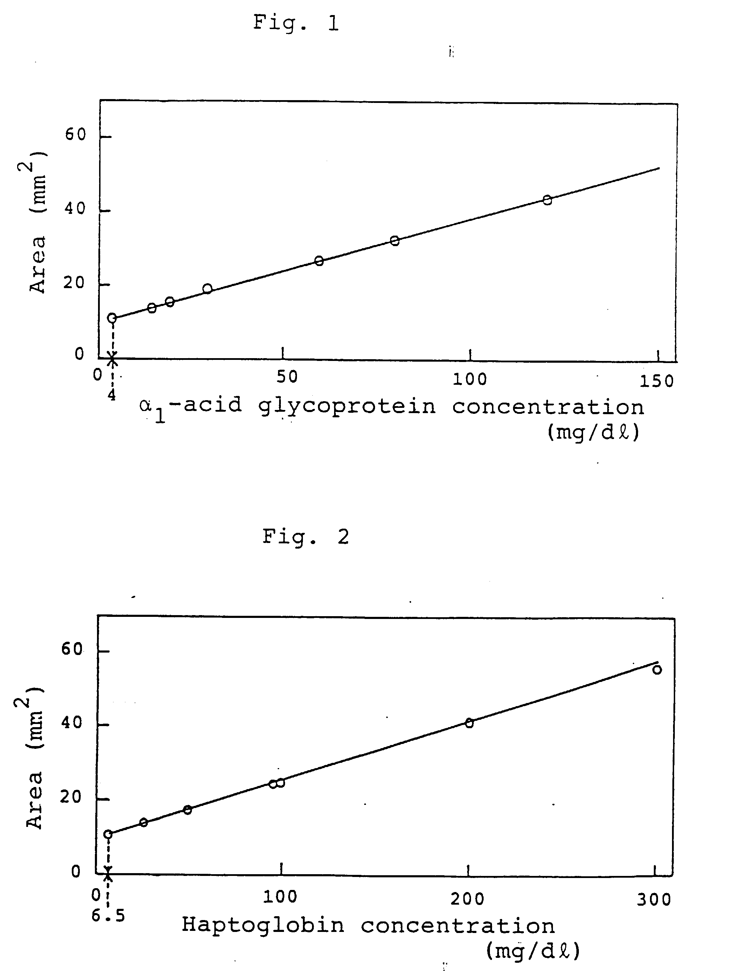

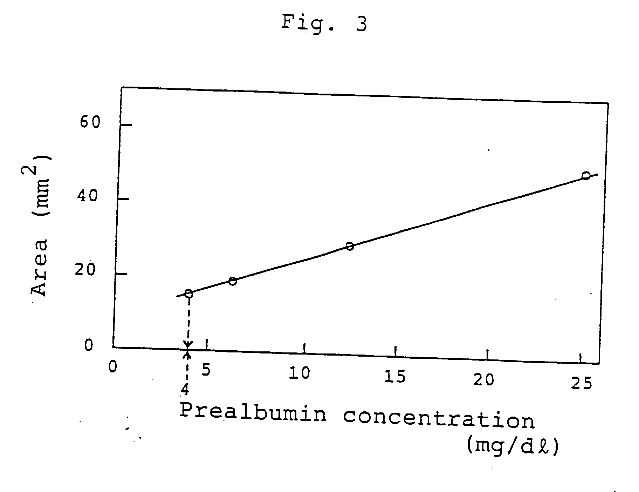

Figures 1 to 3 each shows a standard curve obtained by primary immunodiffusion against α₁-acid glycoprotein, haptoglobin, and prealbumin, respectively.

### DETAILED DESCRIPTION OF THE INVENTION

Albumin, which is the main ingredient of the preparation according to the present invention and also a starting material to be used in the process according to the present invention, is not particularly limited in its origin and includes albumin obtained from mammals, e.g., humans, cattle, rabbits, etc., with human-origin albumin being preferred. The starting material for preparing albumin includes the Fraction V obtained by Cohn's cold alcohol fractionation.

The albumin preparation of the present invention can be obtained by subjecting an albumin-containing aqueous solution to a step of removing a polymer-forming factor present in the solution and then subjecting the solution to a heat treatment. The albumin-containing aqueous solution is usually adjusted to have an albumin content of from 0.1 to 30% (weight/volume, hereinafter the same unless otherwise specified), preferably from about 1 to 10%.

Once it is known that the polymer-forming factor (e.g., haptoglobin) and α₁-AGP which have an isoelectric point lower than that of albumin, various technique capable of removing these proteins from the albumin-containing aqueous solution can be used. Examples of suitable techniques include ion exchange separation using an anion exchanger and affinity chromatography. Haptoglobin, the main component of the polymer-forming factor, is hardly separated from albumin by fractionation because of its relative similarity to albumin in physicochemical properties.

In the case of using an anion exchanger, any insoluble carrier, e.g., dextran (Sephadex®, etc.) agarose (Sepharose®, etc.) cellulose (Cellulofine®, etc.), polyacrylamide, vinylpolymer (Toyopearl®, etc.) having an anion exchange group (e.g., dietylaminoethyl (DEAE) group, quarternarized amino ethyl group (QAE)) which is commonly employed in the art can be used. Specific examples of commercially available anion exchangers are DEAE-Sephadex®, QAE-Sephadex®, DEAE-Sepharose® and Q-Sepharose® (produced by Pharmacia); DEAE-Toyopearl® and QAE-Toyopearl® (produced by Tosoh); A200 Cellulofine® (produced by Seikagaku Kogyo Co., Ltd.); and anion exchange resins. From the standpoint of polymer-forming factor removal efficiency, strong anion exchangers, e.g., Q-Sepharose® and QAE-Toyopearl®, are preferred.

Removal of the polymer-forming factor by using an anion exchanger can be effected by bringing the albumin-containing aqueous solution into contact with the anion exchanger. The amount of the anion exchanger to be used is selected appropriately depending on the polymer-forming factor content in the albumin aqueous solution, exchange capacity of the anion exchanger, and the like, usually ranging from 2 to 5 mℓ, particularly about 3 mℓ, per gram of albumin. The contact may be carried out either in a column system (ion exchange chromatography) or in a batch system, with the latter being preferred in view of removal efficiency.

Where a column system is employed, an albumin aqueous solution is adjusted so as to have a pH between 4.9 and 5.5, more preferably 5.1, and a salt concentration of from 0.001 to 0.2M, preferably from 0.001 to 0.05M. The thus adjusted albumin aqueous solution is passed through a column packed with an anion exchanger equilibrated with an eluent, for example, 0.02M sodium acetate (pH = 5.1). The column is then developed with the eluent, and the non-adsorbed fraction is collected. In order to prevent denaturation of albumin, these procedures are preferably carried out at low temperatures, usually 10°C or lower, not less than 0°C.

Where a batch system is employed, an anion exchanger is added to an albumin aqueous solution having been adjusted in the same manner as described above, and the system is mixed at a temperature of 10°C or less for about 30 minutes to 2 hours. The anion exchanger is then separated by appropriate means, such as centrifugation, and the supernatant liquor is recovered.

In the case of utilizing affinity chromatography, the carrier to be used is an insoluble carrier to which a substance exhibiting specific affinity for the polymer-forming factor, e.g., haptoglobin, is immobilized (hereinafter referred to as affinity carrier). Examples of suitable insoluble carriers are cellulose, agarose and dextran. The substance having specific affinity for the polymer-forming factor, e.g., haptoglobin, is selected according to the kind of the contaminating proteins. For example, an anti-haptoglobin antibody or hemoglobin is used for haptoglobin, and an anti-α₁-AGP antibody is used for α₁-AGP. These antibodies, e.g., an anti-haptoglobin antibody and an anti-α₁-AGP antibody, can be prepared by known antibody production techniques. Bonding between the substance having a specific affinity and the insoluble carrier can be carried out in a usual manner. For example, agarose, e.g., Sepharose®, activated by cyanogen bromide is swollen, and a substance having a specific affinity is coupled with the insoluble carrier in a basic buffer, followed by thoroughly washing with a buffer to obtain an affinity carrier.

Affinity chromatography is performed by contacting an albumin-containing aqueous solution containing the polymer-forming factor, e.g., haptoglobin, with the affinity carrier. For instance, the albumin-containing aqueous solution may be contacted with an affinity carrier to which a plurality of substances each having a specific affinity for the respective contaminating protein are immobilized; or it may be first contacted with an affinity carrier to which a substance having a specific affinity for haptoglobin is immobilized and then with an affinity carrier to which a substance having a specific affinity for α₁-AGP is immobilized, if desired, followed by contact with other affinity carriers having specific affinities for other contaminating proteins.

The amount of the affinity carrier to be used ranges from 2 to 5 mℓ, usually about 4 mℓ, per gram of albumin, though depending on the content of the polymer-forming factor, e.g., haptoglobin, in the albumin-containing aqueous solution and adsorption capacity of the affinity carrier. From the viewpoint of efficiency in removal of the polymer-forming factor, it is preferable to carry out affinity chromatography in a column. For example, the albumin-containing aqueous solution is adjusted to a pH of from about 4.0 to 9.0, preferably from 5.0 to 8.0, more preferably 6.8, and passed through a column packed with the above-described affinity carrier having been equilibrated with a solvent used for dissolving albumin. If desired, the column is washed with the solvent for dissolving albumin to recover the non-adsorbed fraction. To inhibit denaturation of albumin, these procedures are preferably conducted at low temperatures (usually 10°C or less).

The contaminating proteins can thus be removed to obtain an albumin-containing aqueous solution having its polymer-forming factor content reduced.

The albumin-containing aqueous solution having the thus reduced content of the polymer-forming factor is adjusted to have an appropriate concentration and formulated into any desired dose form, for example, charged into vials, followed by a heat treatment for deactivation of viruses. The heat treatment is generally given to an aqueous solution having an albumin concentration of from about 5 to 30% (w/v), usually about 5% (w/v) or from about 20 to 25% (w/v). The heat treatment is carried out under temperature and time conditions selected so as to sufficiently deactivate viruses, for example, at 50 to 70°C, preferably about 60°C, for 5 to 20 hours, preferably about 10 hours. If desired, a stabilizer for albumin, e.g., sodium N-acetyltryptophan and sodium caprylate, may be added either alone or as a mixture thereof to the albumin-containing aqueous solution to be heat-treated. The albumin stabilizer is added in an amount of from about 20 to 60 mg, preferably about 40 mg, per gram of albumin.

The thus obtained albumin preparation contains not more than 3% by weight of polymers based on albumin and contains not more than a detectable limit of α₁-AGP based on albumin.

The albumin preparation according to the present invention can be used at the same dose in the same manner as for the conventional albumin preparations.

The albumin preparations of the present invention have the contaminating viruses deactivated by a heat treatment and the content of polymers and α₁-AGP markedly reduced and are, therefore, excellent in safety, stability and the like.

According to the process of the present invention, viruses which may be incorporated into the preparations can be deactivated by a heat treatment, and since heat-labile contaminating proteins which form polymers on heat treating (polymer-forming factor), e.g., haptoglobin, have been removed before the heat treatment, the resulting albumin preparations have reduced content of polymers and contaminating proteins.

The present invention is now illustrated in greater detail by way of the following Examples, but it should be understood that the present invention is not deemed to be limited thereto.

### EXAMPLE 1

Fraction V of plasma obtained by Cohn's cold alcohol fractionation was dealcoholized with acetone to obtain an acetone-dried powder, which was then dissolved in water to prepare an aqueous solution having an albumin concentration of 10%. After adjusting the pH to 5.1 with 10% (v/v) acetic acid, the solution was passed through a column of DEAE-Sephadex® equilibrated with 0.02 M sodium acetate (pH 5.1) at 4°C. To the effluent were added 20 mM of sodium N-acetyltryptophan and 20 mM of sodium caprylate, and the solution was poured into a vial and heat-treated at 60°C for 10 hours to obtain an albumin preparation.

The polymer content in the resulting preparation was determined by gel chromatography through 4 replicate runs and was found to be 3.0% or less (relative percent by weight based on the albumin content, hereinafter the same with respect to polymer content).

For comparison, an albumin preparation was prepared in the same manner as described above, except that the treatment with DEAE-Sephadex was not conducted. As a result, the polymer content of the resulting preparation was found to be 6.3% (an average of the results of four replicate runs).

Then, the substance adsorbed onto DEAE-Sephadex® was eluted with 5 M magnesium chloride solution at room temperature, and the eluate was analyzed by gel chromatography and cellulose acetate electrophoresis. As a result, the adsorbed substance was found to be proteins mainly comprising haptoglobin.

### EXAMPLE 2

Cyanogen bromide-activated Sepharose® 4B (5.8 g) was swollen with 1 mM hydrochloric acid for 15 minutes and washed with water and then with a basic buffer [containing 0.1M sodium hydrogencarbonate and 0.5M sodium chloride (pH = 8.3)]. In 40 mℓ of the same basic buffer was dissolved 100 mg of human hemoglobin, and 20 mℓ of the above obtained swollen gel was added thereto, followed by stirring for 2 hours. Then, 15 mℓ of 1M glycine (pH = 8.0) was added thereto, and the system was stirred at 4°C for 12 hours, followed by filtration. The gel collected was thoroughly washed with the same basic buffer as used above and equilibrated with a phosphoric acid-buffered sodium chloride aqueous solution to prepare human hemoglobin-immobilized Sepharose.

Separately, the acetone-dried powder of the Fraction V as used in Example 1 was dissolved in water to prepare a 10% albumin aqueous solution, and the solution was adjusted to a pH of 6.8 with sodium carbonate and sodium hydroxide. The solution (200 mℓ) was passed through a column packed with 20 mℓ of the above prepared Sepharose gel at 4°C, and an effluent was collected in 20 mℓ fractions. Haptoglobin in each of fraction Nos. 2 to 8 was determined by primary immunodiffusion (Mancini test described in Mancini et al., Immunochemistry, Vol. 2, No. 3, pp. 295-254, 1985). Further, each fraction sample (5 mℓ) was concentrated to 2 mℓ by means of Centricut-50, and 280 µℓ of a stabilizer solution containing 36 mg of sodium N-acetyltryptophan and 24 mg of sodium caprylate per mℓ was added to the concentrate, followed by heating at 60°C for 10 hours to obtain an albumin preparation. The polymer content in the preparation was determined by gel chromatography. The haptoglobin (Hp) concentration of the effluent and the polymer content of the albumin preparation obtained therefrom are shown in Table 1 below.

**TABLE 1**

| Fraction No. | Hp Concentration | Polymer content |
|---|---|---|
| | (mg/dℓ) | (%) |
| 2 | 0 | 0.94 |
| 3 | 41.8 | 0.82 |
| 4 | 267.4 | 2.70 |
| 5 | 377.1 | 2.87 |
| 6 | 367.9 | 3.12 |
| 7 | 366.9 | 3.13 |
| 8 | 392.6 | 3.01 |

As can be seen from the results of Table 1, the lower the haptoglobin concentration in the effluent, the lower the polymer content of the resulting preparation. Taking the results of Examples 1 and 2 into account, it was proved that the polymer content can be reduced by removing contaminating proteins having an isoelectric point lower than that of albumin (mainly comprising haptoglobin).

### EXAMPLE 3

The Fraction V obtained by Cohn's cold alcohol fractionation (300 g; albumin content: 110.2 g) was dissolved in 1.2 ℓ of cold germ-free distilled water, followed by stirring for about 1 hour. After adjusting to a pH of 4.6 with a 10 v/v% acetic acid aqueous solution, the solution was filtered (pore size: 0.45 µm) at about -2°C. To the filtrate was further added 1.2 ℓ of cold germ-free distilled water, and the solution was adjusted to a pH of 5.1 with 0.8M sodium hydrogencarbonate to prepare an albumin aqueous solution.

Separately, 350 mℓ of QAE-Toyopearl® was packed in a column, thoroughly washed with 500 mℓ of 0.5M sodium chloride, and equilibrated with 0.02M sodium acetate (pH = 5.1) to prepare an anion exchanger column. The above prepared albumin aqueous solution was passed through the column, and the column was washed with 1.2 ℓ of 0.02M sodium acetate. The effluent and the washing were combined, adjusted to a pH of 6.2 with 1N sodium hydroxide, and subjected to concentration with a Pellicon cassette system to give a total amount of 330 mℓ (albumin concentration: about 28%) (hereinafter referred to as adjusted albumin solution).

To the adjusted albumin solution was added 39.6 mℓ of a stabilizer solution containing 5.55 g of sodium N-acetyltryptophan and 3.89 g of sodium caprylate per 100 mℓ, and the solution was adjusted to a pH of 6.85 with 0.1N sodium hydroxide, followed by sterilization by filtration. After adjusting the albumin concentration to 25%, a prescribed amount of the solution was poured into a vial and heat treated at 60°C for 10 hours to obtain an albumin preparation.

The polymer content in the resulting preparation was found to be 1.99% by gel chromatography.

For comparison, an albumin preparation was obtained in the same manner as described above, except that the treatment with the QAE-Toyopearl column was not conducted. The polymer content after the heat treatment was found to be 6.49%, revealing that the polymer content of the albumin preparation according to the present invention is markedly lower than that of the comaprative preparation.

Further, the contents of contaminating proteins in the adjusted albumin solution and the albumin preparation of the present invention were determined by primary immunodiffusion method (Mancini test) using a gel for primary immunodiffusion prepared using anti-α₁-AGP, anti-haptoglobin or anti-prealbumin as an antibody. The results obtained are shown in Table 2 below. The anti-α₁-AGP serum, anti-haptoglobin serum, and anti-prealbumin serum used were prepared from immunized rabbits in a usual manner. A standard curve of precipitated ring area formed by the reaction between each of the anti-serum and the corresponding contaminating protein is shown in Figs. 1 to 3.

As is shown in Table 2, the adjusted albumin solution and the albumin preparation according to the present invention have extremely reduced contents of contaminating proteins. As is obvious from Figs. 1 to 3, the detectable limits of haptoglobin, α₁-AGP, and prealbumin were 6.5 mg/dℓ, 4 mg/dℓ, and 4 mg/dℓ, respectively.

**TABLE 2**

| Contaminating Protein | Content | |
|---|---|---|
| | Adjusted Albumin Solution | Albumin Preparation |
| Haptoblobin | below detectable limit | below detectable limit |
| α₁-AGP | " | " |
| Prealbumin | " | " |

## Claims (Claims for the following Contracting State(s): BE, CH, LI, DE, FR, GB, IT, NL, SE)

1. A process for preparing an albumin preparation in which a polymer-forming factor comprising proteins having an isoelectric point lower than that of albumin and mainly comprising haptoglobin is removed which comprises subjecting a serum albumin aqueous solution to a step removing said polymer forming factor present in the solution, said step being carried out by at least one of ion exchange separation at a pH between 4.9 to 5.5 using an ion exchanger and affinity chromatography, and then subjecting the solution to a heat treatment sufficient to inactivate virus.

2. A process as claimed in claim 1, wherein said ion exchange separation or affinity chromatography is carried out in a column system.

3. A process as claimed in claim 1, wherein an anion exchanger is used.

4. A process as claimed in claim 1, wherein affinity chromatography using a substance having a specific affinity for the polymer-forming factor is employed.

5. A process as claimed in claim 4, wherein the specific affinity substance comprises one or more substances having specific affinity for haptoglobin and α₁-AGP.

6. A process as claimed in claim 2 carried out in a batch system.

7. An albumin preparation obtainable by the process according to any one of claims 1 to 6.

8. An albumin preparation as claimed in claim 7, wherein the polymer content is not more than 3% by weight based on serum albumin content and the α₁-AGP content is less than 4 mg/dl in a 25% adjusted serum albumin solution.

9. An albumin preparation having a haptoglobin content of less than 6.5 mg/dl, an α₁-AGP content of less than 4 mg/dl and a prealbumin content of less than 4 mg/dl in a 25% adjusted serum albumin solution.

## Claims (Claims for the following Contracting State(s): ES)

1. A process for preparing an albumin preparation in which a polymer-forming factor comprising proteins having an isoelectric point lower than that of albumin and mainly comprising haptoglobin is removed which comprises subjecting a serum albumin aqueous solution to a step removing said polymer forming factor present in the solution, said step being carried out by at least one of ion exchange separation at a pH between 4.9 to 5.5 using an ion exchanger and affinity chromatography, and then subjecting the solution to a heat treatment sufficient to inactivate virus.

2. A process as claimed in claim 1, wherein said ion exchange separation or affinity chromatography is carried out in a column system.

3. A process as claimed in claim 1, wherein an anion exchanger is used.

4. A process as claimed in claim 1, wherein affinity chromatography using a substance having a specific affinity for the polymer-forming factor is employed.

5. A process as claimed in claim 4, wherein the specific affinity substance comprises one or more substances having specific affinity for haptoglobin and α₁-AGP.

6. A process as claimed in claim 2 carried out in a batch system.

## Patentansprüche (Patentansprüche für folgende(n) Vertragsstaat(en): BE, CH, LI, DE, FR, GB, IT, NL, SE)

1. Verfahren zur Herstellung eines Albuminpräparates, bei dem ein Polymerbildungsfaktor, der Proteine mit einem geringeren isoelektrischen Punkt als dem von Albumin und hauptsächlich Haptoglobin umfaßt, entfernt wird, umfassend die Entfernung des Polymerbildungsfaktors, der in der Lösung vorhanden ist, aus der wässrigen Serumalbuminlösung, wobei dieser Schritt durch mindestens eine Ionenaustauschertrennung bei einem pH zwischen 4,9 bis 5,5 unter Verwendung eines Ionenaustauschers und Affinitätschromatographie durchgeführt wird, und mit anschließender Wärmebehandlung der Lösung, die für die Virusinaktivierung ausreichend ist.

2. Verfahren nach Anspruch 1, worin die Ionenaustauschertrennung oder Affinitätschromatographie in einem Säulensystem durchgeführt wird.

3. Verfahren nach Anspruch 1, worin ein Anionenaustauscher verwendet wird.

4. Verfahren nach Anspruch 1, worin Affinitätschromatographie unter Verwendung einer Substanz mit spezifischer Affinität für den Polymerbildungsfaktor verwendet wird.

5. Verfahren nach Anspruch 4, worin die spezifische Affinitätssubstanz ein oder mehrere Substanzen mit spezifischer Affinität für Haptoglobin und α₁-AGP umfaßt.

6. Verfahren nach Anspruch 2, das in einem Batch-System durchgeführt wird.

7. Albuminpräparat, erhältlich durch das Verfahren gemäß einem der Ansprüche 1 bis 6.

8. Albuminpräparat nach Anspruch 7, worin der Polymergehalt nicht mehr als 3 Gew.-%, basierend auf dem Serumalbumingehalt, ist und der α₁-AGP-Gehalt weniger als 4 mg/dl in einer 25 %igen eingestellten Serumalbuminlösung ist.

9. Albuminpräparat mit einem Haptoglobingehalt von weniger als 6,5 mg/dl, einem α₁-AGP-Gehalt von weniger als 4 mg/dl und einem Präalbumingehalt von weniger als 4 mg/dl in einer 25 %igen eingestellten Serumalbuminlösung.

## Patentansprüche (Patentansprüche für folgende(n) Vertragsstaat(en): ES)

1. Verfahren zur Herstellung eines Albuminpräparates, bei dem ein Polymerbildungsfaktor, der Proteine mit einem geringeren isoelektrischen Punkt als dem von Albumin und hauptsächlich Haptoglobin umfaßt, entfernt wird, umfassend die Entfernung des Polymerbildungsfaktors, der in der Lösung vorhanden ist, aus der wässrigen Serumalbuminlösung, wobei dieser Schritt durch mindestens eine Ionenaustauschertrennung bei einem pH zwischen 4,9 bis 5,5 unter Verwendung eines Ionenaustauschers und Affinitätschromatographie durchgeführt wird, und mit anschließender Wärmebehandlung der Lösung, die für die Virusinaktivierung ausreichend ist.

2. Verfahren nach Anspruch 1, worin die Ionenaustauschertrennung oder Affinitätschromatographie in einem Säulensystem durchgeführt wird.

3. Verfahren nach Anspruch 1, worin ein Anionenaustauscher verwendet wird.

4. Verfahren nach Anspruch 1, worin Affinitätschromatographie unter Verwendung einer Substanz mit spezifischer Affinität für den Polymerbildungsfaktor verwendet wird.

5. Verfahren nach Anspruch 4, worin die spezifische Affinitätssubstanz ein oder mehrere Substanzen mit spezifischer Affinität für Haptoglobin und α₁-AGP umfaßt.

6. Verfahren nach Anspruch 2, das in einem Batch-System durchgeführt wird.

## Revendications (Revendications pour l'(les) Etat(s) contractant(s) suivant(s): BE, CH, LI, DE, FR, GB, IT, NL, SE)

1. Procédé de préparation d'une préparation d'albumine dans lequel un facteur formant des polymères et comprenant des protéines ayant un point isoélectrique inférieur à celui de l'albumine et comprenant essentiellement de l'haptoglobine est séparé, lequel procédé comprend la soumission d'une solution aqueuse de sérumalbumine à une étape de séparation dudit facteur formant des polymères présent dans la solution, ladite étape étant mise en .uvre par au moins une séparation par échange d'ions à un pH compris entre 4,9 et 5 au moyen d'un échangeur d'anions et de la chromatographie d'affinité, puis la soumission de la solution à un traitement thermique suffisant pour inactiver les virus.

2. Procédé selon la revendication 1, où ladite séparation par échange d'ions ou ladite chromatographie d'affinité est mise en .uvre dans un système de colonne.

3. Procédé selon la revendication 1, où est utilisé un échangeur d'anions.

4. Procédé selon la revendication 1, où est employée la chromatographie d'affinité utilisant une substance ayant une affinité spécifique pour le facteur formant des polymères.

5. Procédé selon la revendication 4, où la substance à affinité spécifique comprend une ou plusieurs substances ayant une affinité spécifique pour l'haptoglobine et l'α₁-AGP.

6. Procédé selon la revendication 2, lequel est mis en .uvre dans un système par lots.

7. Préparation d'albumine susceptible d'être obtenue par le procédé selon l'une quelconquc des revendications 1 à 6.

8. Préparation d'albumine selon la revendication 7, où la teneur en polymères est inférieure ou égale à 3 % en poids par rapport à la teneur en sérumalbumine et où la teneur en α₁-AGP est inférieure à 4 mg/dl dans une solution de sérumalbumine ajustée à 25 %.

9. Préparation d'albumine ayant une teneur en haptoglobine inférieure à 6,5 mg/dl, une teneur en α₁-AGP inférieure à 4 mg/dl et une teneur en préalbumine inférieure à 4 mg/dl dans une solution de sérumalbumine ajustée à 25 %.

## Revendications (Revendications pour l'(les) Etat(s) contractant(s) suivant(s): ES)

1. Procédé de préparation d'une préparation d'albumine dans lequel un facteur formant des polymères et comprenant des protéines ayant un point isoélectrique inférieur à celui de l'albumine et comprenant essentiellement de l'haptoglobine est séparé, lequel procédé comprend la soumission d'une solution aqueuse de sérumalbumine à une étape de séparation dudit facteur formant des polymères présent dans la solution, ladite étape étant mise en .uvre par au moins une séparation par échange d'ions à un pH compris entre 4,9 et 5 au moyen d'un échangeur d'anions et de la chromatographie d'affinité, puis la soumission de la solution à un traitement thermique suffisant pour inactiver les virus.

2. Procédé selon la revendication 1, où ladite séparation par échange d'ions ou ladite chromatographie d'affinité est mise en .uvre dans un système à colonne.

3. Procédé selon la revendication 1, où est utilisé un échangeur d'anions.

4. Procédé selon la revendication 1, où est employée la chromatographie d'affinité utilisant une substance ayant une affinité spécifique pour le facteur formant des polymères.

5. Procédé selon la revendication 4, où la substance à affinité spécifique comprend une ou plusieurs substances ayant une affinité spécifique pour l'haptoglobine et l'α₁-AGP.

6. Procédé selon la revendication 2, lequel est mis en .uvre dans un système par lots.
